# EUROPÄISCHE PATENTSCHRIFT

(11) **EP 2 034 911 B1**
(45) Veröffentlichungstag und Bekanntmachung des Hinweises auf die Patenterteilung: **13.10.2010**
(21) Anmeldenummer: 07726111.3
(22) Anmeldetag: 21.06.2007
(51) Int. Cl.: A61B 17/34, A61B 5/00

(54) **BIEGEWEICHE VORRICHTUNG ZUM EINBRINGEN EINER MEDIZINISCHEN VORRICHTUNG IN DEN KÖRPER**
FLEXIBLE DEVICE FOR INTRODUCING A MEDICAL APPARATUS INTO THE BODY
DISPOSITIF FLEXIBLE DESTINÉ À L'INTRODUCTION D'UN DISPOSITIF MÉDICAL DANS LE CORPS

(30) Priorität: 22.06.2006 EP 06012816
(43) Veröffentlichungstag der Anmeldung: 18.03.2009
(73) Patentinhaber: Roche Diagnostics GmbH, 68305 Mannheim (DE); F. Hoffmann-La Roche AG, 4070 Basel (CH)
(72) Erfinder: LIST, Hans, 64754 Hesseneck-Kailbach (DE); FUERST, Otto, 68519 Viernheim (DE); HAAR, Hans-Peter, 69168 Wiesloch (DE); HAUETER, Ulrich, CH-3506 Grosshöchstetten (CH); HARTTIG, Herbert, 67434 Neustadt (DE); PILL, Johannes, 69181 Leimen (DE)
(74) Vertreter: Wildschütz, Sabine
(86) Internationale Anmeldenummer: PCT/EP2007/005468
(87) Internationale Veröffentlichungsnummer: WO 2007/147591

(56) Entgegenhaltungen:
- EP-A- 1 459 691
- WO-A-03/080169
- WO-A-2004/052594
- WO-A2-2005/044116
- DE-A1- 4 243 715
- US-A- 4 580 551
- US-A- 4 799 495
- US-A- 5 441 499
- US-A- 5 919 199
- US-A- 6 102 886
- US-A1- 2001 047 138
- US-A1- 2005 075 644
- US-B1- 6 743 239

## Beschreibung

### Gebiet der Erfindung:

Die Erfindung betrifft eine Vorrichtung zum Einbringen einer medizinischen Vorrichtung in einen Körper, deren Beweglichkeit vor und nach dem Einstich unterschiedlich ist.

Vorrichtungen zum Einstechen finden im Bereich der Medizin vielfältige Anwendung, insbesondere für transkutane oder subkutane Applikationen. Im diagnostischen Umfeld finden vorwiegend Nadeln und Messer Einsatz, die dazu geeignet sind eine kleine und nicht zu tiefe Hautöffnung zu generieren. Da sie ausschließlich zum Einstich dienen, sind sie meist massiv und starr aufgebaut. Im therapeutischen Bereich finden vorzugsweise Kanülen Einsatz, die dadurch charakterisiert sind, dass sie innen hohl sind und deshalb einen Fluss von Fluiden ermöglichen.

### Stand der Technik:

Bei einer Vielzahl therapeutischer oder diagnostischer Anwendungen ist es erforderlich, eine Vorrichtung, wie beispielsweise eine Kanüle, über einen länger andauernden Zeitraum im Körpergewebe zu implantieren. Dies ist oftmals notwendig, um zum Beispiel eine wiederholte oder lang andauernde Verabreichung therapeutischer oder diagnostischer Fluide zu ermöglichen. Dabei tritt das Problem auf, dass die Vorrichtung eine gewisse Festigkeit aufweisen muss, um ein Einstechen zu ermöglichen und andererseits flexibel sein soll, um Verletzungen bei Bewegung des Patienten zu vermeiden.

Aus den Dokumenten US 20060100582 sowie US 20060100583 sind Katheter bekannt, die zum Einbringen einer Kanüle in den Körper dienen, um beispielsweise Medikamente zu applizieren. Dabei besteht der Katheter aus einem steifen bzw. harten Material zum Einstechen in den Körper. Der einstechende, spitze Teil des Katheters wird nach dem Einstich in den Körper entfernt, um Verletzungen zu vermeiden. Die Kanüle kann aus einem weichen Material bestehen, damit sie sich Bewegungen anpassen kann.

Die Katheter aus US 20060100582 sowie US 20060100583 weisen dabei den Nachteil auf, dass der Katheter ausschließlich zum Einstechen in den Körper dient und nach Einbringen in den Körper keine weitere Funktion übernimmt. Umgekehrt ist die Kanüle nicht zum Einstechen geeignet, da sie aus einem Material besteht, das sich in seiner Form dem Köper anpasst und den Kräften eines Einstichs nicht angepasst ist. Durch die nicht variierbare Härte des Katheters und der Kanüle werden zum Einbringen einer Vorrichtung in einen Körper mindestens zwei Elemente benötigt. Ein Element zum Einstechen und ein weiteres Element zur medizinischen Behandlung.

Aufgabe der Erfindung ist es, eine Vorrichtung zum Einbringen einer medizinischen Vorrichtung (z.B. einer Kanüle oder einem Sensor) in einen Körper bereitzustellen, die einfach zu handhaben ist und sowohl eine problemlose Penetration des Körpers ermöglicht als auch Verletzungen während der Tragezeit im Körper reduziert.

Diese Aufgabe wird durch den Gegenstand von Anspruch 1 gelöst.

Die Aufgabe wird durch die erfindungsgemäße Vorrichtung gelöst. Die Erfindung beschreibt eine Vorrichtung zum Einbringen einer medizinischen Vorrichtung in einen Körper, die im weiteren als Einbringvorrichtung bezeichnet wird, mit einem distalen Bereich, der einen Spitzenbereich zum Erzeugen einer Hautöffnung aufweist, und einem Bereich, der mindestens zwei Teilbereiche beinhaltet, die in einem rigiden Zustand mindestens miteinander und mit dem distalen Bereich im wesentlichen starr verbunden sind und die in einem flexiblen Zustand gegeneinander beweglich angeordnet sind.

Mit Hilfe der Einbringvorrichtung kann eine medizinische Vorrichtung in den Körper eingebracht werden, da die Einbringvorrichtung in ihrem rigiden Zustand zum Einstechen in den Körper geeignet ist. Die medizinische Vorrichtung kann sowohl therapeutischer Natur sein, wie beispielsweise eine Kanüle zur Applikation von Therapeutika oder diagnostischer Natur, wie beispielsweise ein Sensor zur Analyse von Analyten in der Körperflüssigkeit. Dabei kann die Einbringvorrichtung mit der medizinischen Vorrichtung so verbunden sein, dass sie nicht von einander getrennt werden brauchen, also eine Einheit bilden. Im Folgenden wird das Einbringen von diagnostischen Vorrichtungen näher beschrieben, bevorzugter weise von Sensoren.

Die Einbringvorrichtung hat bevorzugter weise eine längliche Ausdehnung, um beim Eindringen in die Haut möglichst wenig Schmerz zu verursachen. Weiterhin weist die längliche Vorrichtung im Querschnitt vorzugsweise eine runde Form auf. Die Vorrichtung weist mindestens zwei Bereiche auf. Davon ist ein Bereich der distale Bereich mit einer Nadelspitze. Ein weiterer Bereich ist der Segmentbereich, der wiederum mindestens zwei Teilbereiche beinhaltet. Diese mindestens zwei Teilbereiche sind miteinander und mit dem distalen Bereich verbunden. Die mindestens zwei Teilbereiche, die auch als Segmentbereich bezeichnet werden, können in zwei verschiedenen Zuständen vorliegen. In einem ersten Zustand sind die mindestens zwei Teilbereiche rigide, also starr miteinander verbunden, während sie in einem zweiten, flexiblen Zustand gegeneinander beweglich sind. Da die Teilbereiche auch in ihrem flexiblen Zustand miteinander verbunden sind, können sie sich gegeneinander bewegen. Im flexiblen Zustand kann die Bewegung der Teilbereiche mit der Bewegung einer Schlange verglichen werden, die zwar Teilbereiche ihres Körpers quer zu ihrer Ausrichtung bewegen kann, aber die Reihenfolge der Teilbereiche sich dabei nicht ändert. Der rigide, also starre Zustand kann durch Kompression der Vorrichtung, wie sie beispielsweise beim Einstich stattfindet oder durch ein formgebendes Element bewirkt werden. Beim Einstich der Vorrichtung in den Körper bildet die Oberfläche des Körpers einen Widerstand, der ausreicht, um die Segmente gegeneinander zu drücken und die Vorrichtung in den rigiden oder starren Zustand zu versetzen. Nachdem die Vorrichtung in den Körper eingestochen ist, ist der Widerstand überwunden und es wirken keine Druckkräfte mehr auf die Teilbereiche bzw. Segmente.

Zusätzlich wird der rigide Zustand durch das formgebende Element erreicht. Das formgebende Element ist ein Bindemittel. Dieses Bindemittel ist vorzugsweise mindestens zum Teil wasserlöslich bzw. kann im Körper gelöst oder abgebaut werden. Die mindestens zwei Teilbereiche können wie bei einer Feder aus mehreren Windungen bestehen oder können aus Segmenten verschiedenster Formen, wie beispielsweise Ringe oder Zylinder bestehen.

Kurz vor dem Einstich befindet sich die Einbringvorrichtung in dem rigiden Zustand. In diesem rigiden Zustand ist die Einbringvorrichtung so starr, dass sie, ohne seitlich verbogen zu werden, in den Körper eingestochen werden kann. Nach Eindringen der Einbringvorrichtung in den Körper, wird die Vorrichtung in dem Bereich, der die mindestens zwei Teilbereiche beinhaltet, in den flexiblen Zustand übergeführt. Dies findet passiv durch das Auflösen von dem Fixierungsmittel statt. Die Beweglichkeit der Vorrichtung in eingestochenem Zustand dient dem Komfort des Patienten, der diese Vorrichtung über einen längeren Zeitraum von mehreren Tagen in seinem Körper tragen kann. Durch die beweglichen Teilbereiche ist es der Vorrichtung möglich, sich Bewegungen des Patienten anzupassen, sodass Verletzungen im Gewebe reduziert werden. Ziel ist es, dass das Gewebe um die Vorrichtung herum nicht traumatisiert wird. Neben dem Komfort für den Patienten hat die niedrige Traumatisierung des Gewebes den Vorteil, dass metabolische Vorgänge des Körpers an der Einstichstelle nicht signifikant gegenüber dem Normalzustand geändert werden und somit ein repräsentativer Zustand vorliegt. Dieser ungestörte Gewebszustand ist Voraussetzung für eine reproduzierbare und repräsentative Messung von Analyten in dem Gewebe.

Die medizinische Vorrichtung, die bevorzugter weise mit dem Gewebe in Wechselwirkung tritt, kann mit der Einbringvorrichtung fest verbunden sein, oder es kann eine lose Verbindung zwischen Einbringvorrichtung und medizinischer Vorrichtung vorliegen. Die Verbindung kann hierbei zu einem beliebigen Zeitpunkt vor oder nach dem Einstich gelöst oder hergestellt werden und so die beiden Vorrichtungen von einander getrennt oder zusammen geführt werden. In einer bevorzugten Ausführungsform ist ein Sensor zur Analyse in der Gewebsflüssigkeit fest mit der Einbringvorrichtung verbunden.

Um die Verträglichkeit und den Komfort für den Patienten weiterhin zu erhöhen, kann zusätzlich eine Membran über die mindestens zwei Teilbereiche angebracht werden. Diese Membran soll verhindern, dass Gewebe zwischen die mindestens zwei gegeneinander beweglichen Teilbereiche eingeklemmt wird, wenn die Vorrichtung in ihrem flexiblen Zustand vorliegt. Außerdem kann durch die Membran die Funktionsweise einer in die Vorrichtung eingebrachten medizinischen Vorrichtung gesichert werden, da die Membran auch als Schutz gegen das Eindringen von großen Molekülen benutzt werden kann. Bevorzugter Weise ist diese Membran sehr dünn und außerdem flexibel sowie biokompatibel.

Weiterhin wird eine Ausführungsform des Systems beschrieben, bei der mindestens ein Teil der Vorrichtung hohl ist. Weiterhin kann das System einen Sensor aufweisen. Der Sensor kann sich bevorzugter Weise in dem hohlen Bereich der Vorrichtung befinden. Zum Kontaktieren des Sensors weist die Vorrichtung ein Kontaktelement auf. Wenn der Spitzenbereich hohl ist, kann sich der Sensor auch in dem hohlen Spitzenbereich befinden. Der Sensor besteht dabei aus mindestens zwei Elektroden, die auf ihrem aktiven Bereich mit einem Enzym beschichtet sind, das zum Nachweis des Analyten dient, wie dies aus den US Patenten US 5,997,817 und US 6,814,844 bekannt ist. Die Elektroden werden beispielsweise über das proximale Ende der Vorrichtung kontaktiert, um die Stromversorgung und die Datenermittlung zu gewährleisten. Die Stromversorgung und die Datenermittlung können in das System integriert sein, können aber auch außerhalb der Einbringvorrichtung angeordnet sein und an das System über Kontakte angekoppelt werden.

Aus den elektrischen Daten des Sensors kann in einer Auswerteeinheit die Analytkonzentration berechnet werden, die beispielsweise über ein Display dem Patienten angezeigt wird. Diese Auswerte- und/oder Anzeigeeinheit kann direkt an die Vorrichtung zum Einstehen in einen Körper integriert sein oder an diese je nach Bedarf angekoppelt werden. Die Vorrichtung kann aber auch mit einer Datenübertragungsvorrichtung versehen sein, die die elektrischen Daten an eine Auswerteeinheit übermittelt. Diese Auswerteeinheit kann sich beispielsweise zusammen mit einer Anzeigeeinheit in einem Gerät befinden, welches der Patient an einer anderen Stelle am Körper trägt (bspw. eine Uhr) oder als hand-held Gerät (wie beispielsweise ein Organizer, Mobil-Telefon oder andere elektronischen Kleingeräte) mit sich trägt.

Weiterhin kann die Einstichvorrichtung eine Vorrichtung zum Entfernen der Einstichvorrichtung aus dem Körper des Patienten beinhalten. Dies kann beispielsweise ein Faden, bevorzugter Weise aus Stahl oder Nylon, sein, der an der Einstichvorrichtung angebracht ist. Bevorzugterweise ist diese Vorrichtung mit der Spitze der Einstichvorrichtung verbunden, um keine Gewebsteile in den Zwischenräumen zwischen den Teilbereichen einzuklemmen.

### Spitzenbereich

Der Spitzenbereich befindet sich - wie erwähnt - im distalen Bereich der Vorrichtung. Dieser Bereich dient zum schmerzarmen Einbringen der Vorrichtung in die Haut. Die Seitenwände laufen in diesem Bereich aufeinander zu und münden in einer Spitze. In einer bevorzugten Ausführungsform ist die Spitze für einen atraumatischen Einstich in den Körper geeignet, wie dies bei Akkupunkturnadeln bekannt ist. Das Material dieser Spitze ist dazu geeignet, den Kräften wie sie beim Einstich in einen Körper auftreten zu widerstehen. Dies kann beispielsweise durch Materialien wie Keramik, Metall oder Kunststoff sowie Kombinationen aus diesen gewährleistet werden. Als Metalle eignen sich hierbei vor allem gewebeverträgliche Stähle mit Cr, Ni, CoCr oder Titan Legierungen. Die Keramiken können vorzugsweise auf Aluminiumoxid oder Zirkoniumoxid basieren. Bei der Verwendung von Kunststoffen sind beispielsweise Polysulfone, Polyamide oder PEEK™ besonders geeignet. Die Spitze kann dabei auch innen hohl sein, wie dies bei einer Kanüle der Fall ist. An den Spitzenbereich, der mehrere Millimeter lang sein kann, bevorzugter weise 1 bis 4 mm, schließt sich der flexible Bereich, auch Segmentbereich genannt an, der aus anderen Materialien als die Spitze bestehen kann.

### Teilbereiche / Segmente

Der Segmentbereich beinhaltet mindestens zwei Teilbereiche. Diese Teilbereiche können Segmente enthalten, die aus verschiedenen Materialien oder deren Mischung bestehen können, wie beispielsweise Gewebe, Keramik, Kunststoff oder Metall. Die Segmente können verschiedenste Formen aufweisen, wie beispielsweise die Form einer Feder, bei der die Teilbereiche ohne Unterbrechung in einander übergehen oder Ringe bzw. Zylinder, die plan aufeinander liegen oder Kanten aufweisen, die ineinander haken. Die einzelnen Teilbereiche sind dabei miteinander verbunden, sodass sie zwar beweglich gegeneinander sein können, aber nicht auseinander rutschen. Diese flexible Verbindung kann beispielsweise ein Verhaken der Segmente untereinander sein. Dies entspricht beispielsweise dem Prinzip einer Gliederkette. Die Segmente können dabei innen hohl sein, oder aber auch massiv. Ihre Größe und Ausdehnung kann variieren. Die Länge des Segmentbereiches kann stark variieren, je nach Anwendungsgebiet der Einbringvorrichtung. In einer bevorzugten Ausführungsform ist der Segmentbereich, der zusätzlich noch einen Sensorbereich enthalten kann zwischen 5 und 30 mm lang.

### Membran

Der Segmentbereich kann mit biokompatiblen Beschichtungen überzogen sein oder selbst biokompatibel sein, da er sich für mehrere Tage im Körper eines Patienten befindet. Alternativ oder zusätzlich kann über die Segmente eine Membran gezogen werden, die verschiedene Funktionen (wie beispielsweise die Biokompatibilität) übernehmen kann. Die Membran verhindert das Austreten von Enzymen und anderen Reagenzien aus dem Sensorbereich und verhindert gleichzeitig das Eindringen von Komponenten (wie große Proteine, Zellen oder Zellbestandteilen) aus dem Blut und / oder der Gewebsflüssigkeit in den Sensorbereich. Solche Komponenten können die Haltbarkeit und Funktionsweise des Sensorbereiches stark beeinflussen und sollten deshalb vom Sensorbereich fern gehalten werden. Die Membran, die gegebenenfalls über die beweglichen Teilbereiche gezogen wird, kann aus ausreichend festen aber flexiblen, biokompatiblen Werkstoffen bestehen. Dies sind beispielweise Materialien, die als (Mikro)-Dialysemembranen eingesetzt werden, wie Zellulose (regenerierte) oder Zellulosederivate (darunter z.B. Acetat- oder Diäthylaminoathyl-substituerte Zellulose). Des weiteren sind synthetische Polymere wie z.B. Polysulfone, Polymethacrylat (PMMA), Polyacrylnitril, Polyacrylethersulfone oder Copolymere daraus geeignet, aber auch Membranen auf Basis von Polysiloxanen oder modifizierten Polyurethanen und anderen Polymeren.

### Formgebendes Element

Das formgebende Element kann benutzt werden, um die mindestens zwei Teilbereiche zu fixieren und dadurch starr zu machen. Das formgebende Element kann dabei unterschiedlich ausgestaltet sein. Dabei kann das formgebende Element in mindestens zwei verschiedene Zustände gebracht werden. Bevorzugter weise nimmt das formgebende Element den einen Zustand vor dem Einbringen der Vorrichtung an und den anderen Zustand nach dem Einbringen der Vorrichtung. In dem einen Zustand sind die mindestens zwei Teilbereiche starr und in dem anderen Zustand sind die mindestens zwei Teilbereiche flexibel angeordnet. Ist das formgebende Element massiv ausgestaltet, wie beispielsweise in Form eines Drahtes oder einer Klammer, so kann es die Teilbereiche zu jedem beliebigen Zeitpunkt vor oder nach dem Einbringen der Vorrichtung in den Körper in den starren oder den flexiblen Zustand bringen. Diese Art von formgebenden Elementen wird im Folgenden als Verbindungselement bezeichnet.
Alternativ kann das formgebende Element aber auch in, auf oder zwischen die Teilbereiche eingebracht werden und nach dem Einbringen der Vorrichtung in den Körper seine Gestalt so verändern, dass es die Teilbereiche nicht erneut fixieren kann. Diese Art des formgebenden Elementes kann beispielsweise ein Fixierungsmittel sein.

### Fixierungsmittel

Das Fixierungsmittel, das möglicherweise in, auf oder zwischen den Teilbereichen eingebracht wird, um die Vorrichtung in den rigiden Zustand zu überführen, fixiert mindestens einen Teil der Teilbereiche (wie Segmentbereich, distaler Bereich, proximaler Bereich, Sensorbereich etc.) aneinander. Das Fixierungsmittel weist außerhalb des Körpers eine ausreichende Härte auf, um die Teilbereiche beim Einstich der Vorrichtung in den Körper ausreichend fixieren zu können. In eingestochenem Zustand soll sich das Fixierungsmittel im Körper zumindest zum Teil auflösen und dadurch eine flexible Beweglichkeit der Teilbereiche bzw. Segmente gegeneinander ermöglichen. Bevorzugter weise kann die Veränderung der Beweglichkeit der fixierten Teilbereiche wie folgt hervorgerufen werden:
- Teil- oder Vollauflösung des Fixierungsmittels: hierbei besteht mindestens ein Teil des Fixierungsmittels aus einer niedermolekularen Komponente, welche sich in der Gewebsflüssigkeit bzw. in Wasser auflöst und damit die starre Struktur der fixierten Teilbereiche aufhebt.
- Quellung/Erweichen des Fixierungsmittels: Gewebsflüssigkeit bzw. Wasser quellt das Fixierungsmittel auf oder erweicht es, und das Fixierungsmittel verliert dabei seine Starrheit. Hierzu sind beispielsweise Polymere aus Hydroxymethylmethacrylat (in PHEMA) geeignet. Zur Erweichung werden bevorzugt vernetzte, wasserquellbare Polymere, wie Polyacrylsäuren, oder Stärkederivate sowie Fette oder Paraffine benutzt.
- bio-degradierbare Biopolymere: Hydrolyse empfindliche Polymere, die in trockenem Zustand hohe Stabilität aufweisen und in geeigneter Umgebung (wie in Gewebsflüssigkeit) abgebaut werden. Zu diesen Polymeren gehören beispielsweise Polyglycolsäure, D,L -Polymilchsäure oder deren Derivate und / oder Copolymere.

Diese Methoden und Komponenten können auch miteinander kombiniert werden, um eine möglichst große Verträglichkeit für den Patienten zu erreichen und die Gewebsflüssigkeit möglichst wenig in ihrer Zusammensetzung zu verändern.

Als lösliche Materialien sind bevorzugter Weise Salze einsetzbar, besonders bevorzugt sind kristallisierende Salze, wie NaCl, Na-Lactat oder organische Verbindungen, wie Zucker, die druckfeste Kristalle bilden, die ausreichen, um die Einbringvorrichtung zu versteifen. Alternativ können auch glasartige Materialien, wie z.B. Zuckerglas verwendet werden. Kristallisierende Materialien werden bevorzugt in Form von gesättigten oder übersättigten Lösungen in die Zwischenräume der Einbringvorrichtung eingebracht, wobei durch Verdunsten des Lösungsmittels es zur Bildung der Kristalle in den Zwischenräumen kommt. Als Beispiel einer solchen Zuckerglaslösung dient eine Mischung aus Rohrzucker und Wasser im Verhältnis von 4,9:1. Die Mischung wird erhitzt, bis eine homogene Masse ensteht. Eine Einstichvorrichtung wird mit ihren flexiblen Teilbereichen mindestens zum Teil in die heiße Masse getaucht, überschüssiges Material wird abgestreift.

Die erweichenden Materialien können beispielsweise entweder als Lösung in einem nichtwässrigen Lösungsmittel in die Teilbereiche eingebracht werden, oder eine wasserlösliche Vorstufe des Materials wird in die Teilbereiche eingebracht und darin so vernetzt oder chemisch modifiziert, dass das Material seine Wasserlöslichkeit verliert.

Bestehen die Teilbereiche beispielsweise aus den Windungen eines geflochtenen oder gedrehten Gewebes, so kann in oder auf das Gewebe Fixierungsmittel als stabilisierende Substanz gebracht werden, die die Teilbereiche vor dem Einstich der Vorrichtung in den Körper starr macht und sich nach dem Einbringen der Vorrichtung im Körper wie oben beschrieben auflöst und / oder quillt und die Vorrichtung flexibel gegenüber Bewegungen des Patienten macht. Dieses Fixierungsmittel kann ebenfalls unter, in oder auf eine Membran gegeben werden, wie sie bereits beschrieben wurde, um diese stabilisierende Wirkung zu erzielen. Alternativ könnte als Fixierungsmittel eine Flüssigkeit, wie beispielsweise Wasser benutzt werden, die vor dem Einbringen der Vorrichtung in den Körper vereist wird und sich nach dem Einbringen der Vorrichtung in den Körper dort auflöst bzw. schmilzt.

### Verbindungselement

Wie bereits erwähnt können auch Verbindungselemente zur Fixierung der Teilbereiche eingesetzt werden, die Wasser unlöslich sind und diese Eigenschaft vor und nach dem Einbringen der Vorrichtung in den Körper im Wesentlichen nicht ändern. Dabei können die Teilbereiche beispielsweise durch eine Klammer zusammen gehalten, die je nach Bedarf gespannt oder gelöst werden kann. Alternativ können die Teilbereiche auch durch einen oder mehrere Magnete zusammengezogen werden, deren magnetische Wirkung durch Elektrizität gesteuert werden kann. Eine bevorzugte Ausführungsform des Verbindungselementes ist ein Zugdraht, der zur losen Verbindung der Teilbereiche im flexiblen Zustand und zu einer festen Verbindung der Teilbereiche in dem starren Zustand dient. Um die Teilbereiche starr miteinander zu verbinden, muss der Zugdraht verkürzt bzw. gestrafft werden, was z.B. mechanisch oder elektrisch erreicht werden kann. Bei der mechanischen Verkürzung des Zugdrahtes kann der Zugdraht durch den Patienten selber gespannt werden oder durch ein zusätzliches Element, wie beispielsweise ein Butterfly. Ein Butterfly ist ein Hilfsmittel bei der Injektion von Nadeln, um eine komfortable Handhabung zu gewährleisten, wie dies im Stand der Technik bekannt ist, und in der US Patentanmeldung US 20060100575 beschrieben wird. Hierzu kann der Zugdraht aus einem beliebigen Material gefertigt sein, der eine Straffung (und erneute Lösung) so ermöglicht, dass der Zugdraht nicht reißt. Bei der elektrischen Verkürzung des Zugdrahtes, kann vorzugsweise ein Material eingesetzt werden, das seine Ausdehnung unter Einsatz von Strom verändert. Dies wird vorzugsweise durch eine Metalllegierung, wie beispielsweise aus Ti/Ni, erreicht. Diese Legierung zieht sich unter Einsatz von Strom zusammen und dehnt sich nach Beendigung der Stromzufuhr wieder aus und nimmt seine ursprüngliche, flexible Form wieder an. Ein Zugdraht kann eingesetzt werden, wenn die Teilbereiche aus im Wesentlichen starren Segmenten bestehen. Der Zugdraht kann aus verschiedenen Materialien bestehen, die mit verschiedenen Bereichen der Vorrichtung verbunden werden können. Dies können verschiedene Kunststoffe sein, aber auch Metall und/oder Metalllegierungen.

### Sensor

Der Sensor kann, wie bereits beschrieben, direkt in die Einbringvorrichtung integriert sein oder an diese angekoppelt werden. Der Sensor kann sich an beliebigen Stellen der Einbringvorrichtung befinden, die nach Einbringen der Vorrichtung in den Körper mit Körperflüssigkeit in Kontakt stehen. Wenn er lose mit der Einbringvorrichtung verbunden ist, so sollte wenigstens ein Bereich der Einbringvorrichtung innen hohl sein, um den Sensor einschieben zu können.

Ist der Sensor fest mit der Einbringvorrichtung verbunden, wie dies in einer bevorzugten Ausführungsform der Fall ist, so braucht die Einbringvorrichtung keinen Hohlraum aufzuweisen, sondern der Sensor ist in oder auf der Vorrichtung so angebracht, dass ein ausreichender Flüssigkeitsaustausch mit der Gewebsflüssigkeit stattfinden kann.

In einer bevorzugten Ausführungsform befindet sich der Sensorbereich im Inneren der Einbringvorrichtung. Um einen Flüssigkeitsaustausch des Sensors mit der umgebenden Flüssigkeit zu gewährleisten, können Perforationen in die Wandung der Einbringvorrichtung in eingebracht werden, um einen ausreichenden Flüssigkeitsaustausch mit der Gewebsflüssigkeit zu gewährleisten. Dies hat den Vorteil, dass der Sensor leicht kontaktiert werden kann und vor mechanischen Einflüssen außerhalb der Einbringvorrichtung geschützt ist. Durch ausreichend große Perforationen wird ein schneller Flüssigkeitsaustausch gewährleistet.

Der Sensorbereich kann sich alternativ auf der Außenseite der Einbringvorrichtung befinden und gegebenenfalls mit einer Membran geschützt werden. Die hat den Vorteil, dass der Sensorbereich direkt mit der Flüssigkeit in Kontakt treten kann und der Flüssigkeitsaustausch nicht durch Diffusionsvorgänge (außer durch eine mögliche Membran) verzögert wird.

Der Sensor kann zur Messung physikalischer Zustände wie Temperatur und Druck und/oder chemischer Zustände oder Konzentrationen verwendet werden und auf verschiedenen Messprinzipien beruhen. Bevorzugt sind resistive, amperometrische oder colorimetrische Sensoren. Auch galvanische, kapazitive, induktive oder andere Sensoren sind denkbar. Es ist aber auch möglich über das Einbringen von Lichtleitern eine optische Messung vorzunehmen. Neben einer direkten Detektion des Analyten z.B. auf optischer Basis, ist eine Messung des Analyten mittels eines Reagenz möglich, welches eine Reaktion des Analyten mit dem Reagenz, wie z. B. einem Enzym, voraussetzt. Solche Reaktionen sind hinlänglich im Stand der Technik bekannt, wie in US 4,490,465 und US 20050201897 beschrieben.

Die Stromquelle für den Sensor kann dabei ein, in Teile der Einbringvorrichtung integrierter Akkumulator sein, der induktiv aufgeladen werden kann. Die Stromquelle kann sich jedoch auch außerhalb der Einbringvorrichtung befinden und nur bei Benutzung mit dieser verbunden werden.

Weiterhin kann sich in der Einbringvorrichtung die Elektronik zur Ansteuerung des Sensors befinden. Dies können ein Mikroprozessor zur Steuerung und Datenaufbereitung sowie eine aktive Sendereinheit sein, um komprimierte Daten zu versenden und/oder eine passive Funk-Einheit, wie eine RFID (radio frequency identification) Einheit, zum Datentransfer, wie im Stand der Technik bekannt (US 20060064037). Die DatenVerarbeitung und/oder Versendung kann jedoch auch außerhalb der Einbringvorrichtung vorgenommen werden.

### Figurenbeschreibung:

**Figur 1****:** Darstellung eine Vorrichtung zum Einbringen in den Körper mit Nadelspitze, Sensorbereich, Segmentbereich und proximalem Bereich.
**Figur 2a****:** Vergrößerungsdarstellung des Segmentbereiches aus Figur 1 in rigidem Zustand.
**Figur 2b****:** Darstellung des Segmentbereiches aus Figur 1 in flexiblem Zustand.
**Figur 3****:** Darstellung einer Zugvorrichtung zum Spannen des Segmentbereiches aus Figur 1 in Form eines Butterflys.
**Figur 4****:** Darstellung einer Insertionshilfe.
**Figur 4a****:** Darstellung des proximalen Endes der Vorrichtung nach Insertion in den Körper.

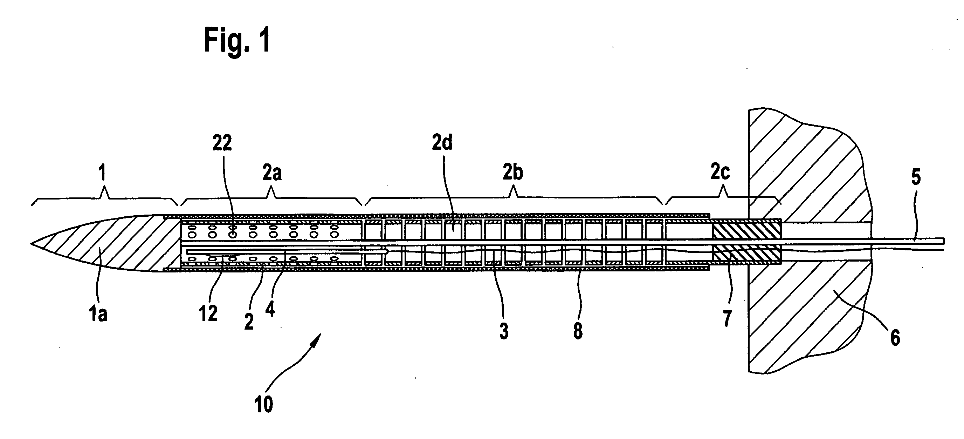

Figur 1 stellt eine Vorrichtung (10) zum Einbringen in einen Körper dar. An dem distalen Ende der Vorrichtung (10) befindet sich der distale Bereich mit (1) einer Nadelspitze (1a). An diese Nadelspitze (1a) schließt sich ein Segmentbereich (2), der aus dem gleichen oder einem anderen Material wie die Nadelspitze (1a) gefertigt sein kann, an. Dieser Segmentbereich (2) beinhaltet im Anschluss an die Nadelspitze (1a) einen Sensorbereich (2a), der in den Segmentbereich (2) eingebracht ist. Dieser Sensorbereich (2a) weist Perforationen (22) in der Hülle auf, um einen Flüssigkeitsaustausch mit dem Gewebe zu gewährleisten. Auf den Sensor (4) sind Reagenzien (12) aufgebracht, die mit dem Analyten reagieren und eine elektrisch messbare Änderung des Signals an den Elektroden hervorrufen. An den Sensorbereich (2a) schließt sich ein mittlerer Segmentbereich (2b) an, der im proximalen Teil der Vorrichtung (10) in einen proximalen Bereich (2c) übergeht. Dieser Bereich (2c) besteht vorzugsweise aus einem glatten Rohr. In diesem proximalen Bereich (2c) befindet sich ein Flüssigkeitsstopp (7) der verhindert, dass Flüssigkeit aus dem mittleren Segmentbereich (2b) in den proximalen Bereich (2c) eindringt. Der Flüssigkeitsstopp besteht bevorzugter weise aus einem Elastomer. Außerdem wird durch diesen Flüssigkeitsstopp (7) verhindert, dass Keime durch den proximalen Bereich (2c) der Vorrichtung (10) in den Segmentbereich (2) bzw. ins Gewebe eindringen können. Die Vorrichtung (10) ist ansonsten zwischen der Nadelspitze (1a) und dem proximalen Bereich (2c) innen hohl. Es befindet sich im Inneren der Vorrichtung (10) eine Zuleitung (3) für den Sensor (4). Der Segmentbereich (2) besteht über seine komplette Länge zwischen Nadelspitze (1a) und proximalem Bereich (2c) aus einzelnen Segmenten (2d). Diese Segmente (2d) grenzen an den Sensorbereich (2). Der Sensorbereich (2), ist mit einem Zugdraht (5) verbunden, welcher durch den Flüssigkeitsstopp (7) geleitet wird und mit weiteren Teilen der Vorrichtung (10) verbunden ist. Um den Segmentbereich (2) herum befindet sich eine für den Analyten durchlässige Membran (8), die die Segmente flexibel umschließt.

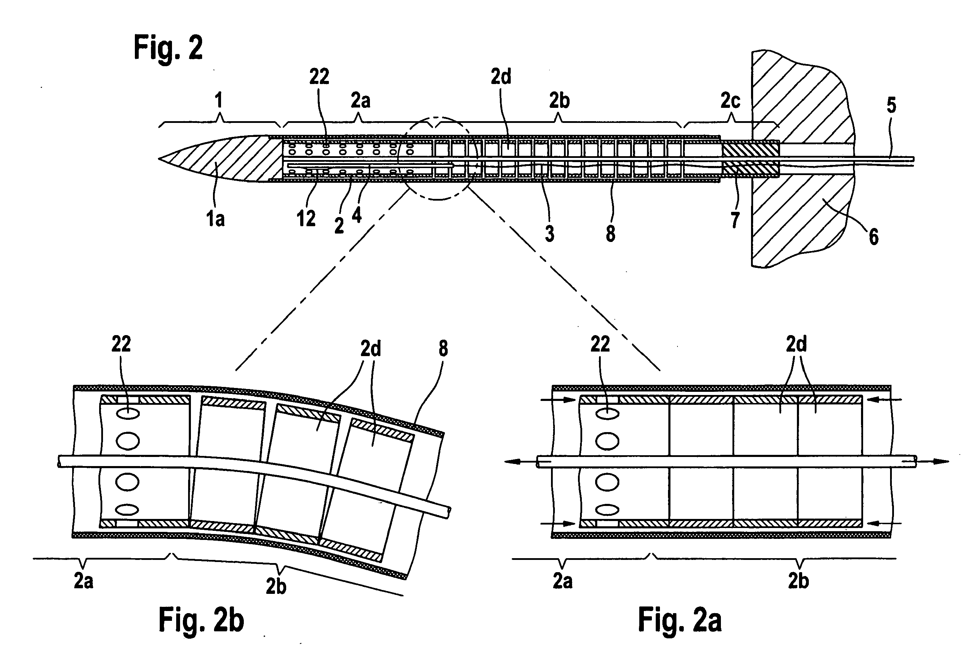

In Figur 2 ist ebenfalls der implantierte Teil der Vorrichtung (10) dargestellt, wobei Figur 2a einen Ausschnitt aus dem Segmentbereich (2) zeigt, der im Übergang vom Sensorbereich (2a) und dem mittleren Segmentbereich (2b) liegt. In Figur 2a ist dieser Segmentbereich (2b) in seinem rigiden Zustand gezeigt. Die Pfeile zeigen an, dass die Segmente (2d) aufeinander gedrückt werden. Dies geschieht durch eine Kraft, die auf den Zugdraht (5) ausgeübt wird, der mit mindestens zwei der Segmente (2d) verbunden ist. Um eine optimale Versteifung der Segmente zu erreichen, ist der Zugdraht (5) mit dem distalen Bereich (1) und dem proximalen Bereich (2c) verbunden. Durch Verkürzung des Zugdrahtes (5) in diesem Bereich, werden die Segmente (2d) aufeinander gedrückt und die Vorrichtung zum Einbringen (10) versteift. In Figur 2b ist der gleiche Segmentbereich dargestellt wie in Figur 2a, wobei der Zugdraht (5) nicht unter Spannung steht, sodass die Segmente (2d) zueinander beweglich sind.

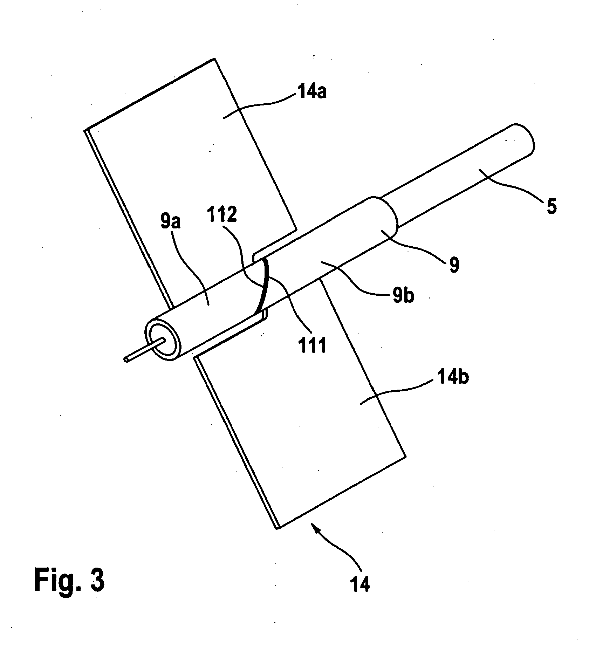

In Figur 3 ist eine Möglichkeit dargestellt, wie der Zugdraht (5) aus Figuren 1 und 2 gespannt werden kann. Hierzu ist ein so genannter Butterfly (14) als Zugvorrichtung vorgesehen. Die beiden Flügel (14a) und Flügel (14b) liegen mit schrägen Flächen, der so genannten ersten Hebeschräge (111) und der zweiten Hebeschräge (112) aufeinander und sind so gegeneinander fixiert, dass sie immer koaxial zueinander stehen.

Am ersten Flügel (14a) ist der Zugdraht (5) befestigt, am zweiten Flügel (14b) der proximale Teil (2c) des Segmentbereiches (2). Werden die Flügel (14a) und (14b) gegeneinander verdreht, so sorgen die Hebeschrägen (111) und (112) dafür, dass die beiden Flügel (14a) und (14b) axial auseinander rücken müssen. Dabei wird der proximale Bereich (2a) des Segmentbereiches nach vorne geschoben, der distale Bereich (1) mit der Spitze (1a) über den Zugdraht (5) nach hinten gezogen. Dadurch gerät der Segmentstapel (2b)unter Druckkraft und die Segmente können nicht mehr gegeneinander verkippen. Damit verhält sich der komplette Mechanismus wie ein starrer Stab.

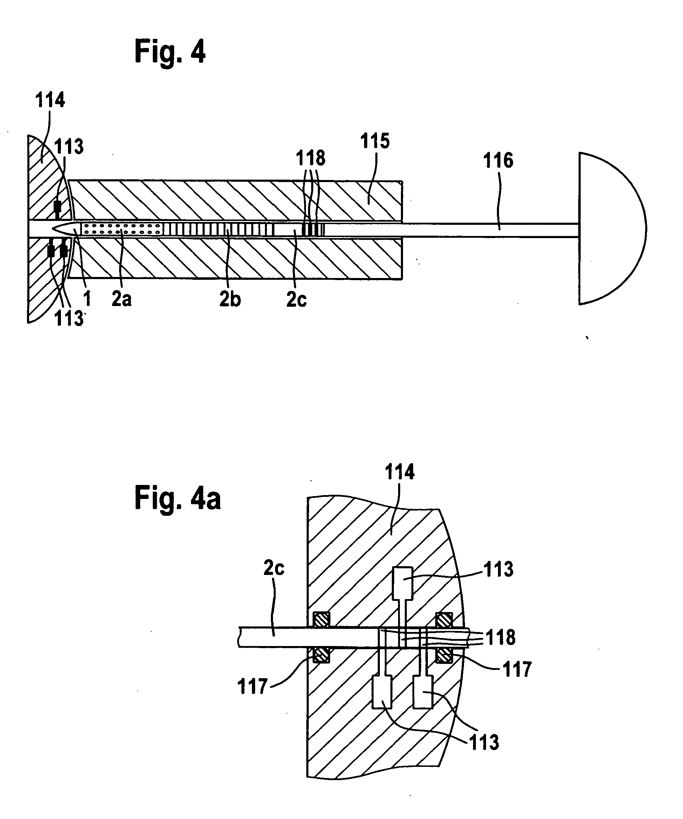

Figur 4 zeigt eine Vorrichtung zum Einbringen in einen Körper (10) mit einem integrierten Sensorbereich (2a), dem Segmentbereich (2) sowie einem Kontaktierungsbereich (118) zum elektrischen Kontaktieren des Sensors. Die Vorrichtung zum Einbringen (10) ist an ihrem proximalen Bereich (2c) lösbar mit einem Applikator (116) verbunden, der zur leichteren Einführung der Vorrichtung (10) dient. Um eine ausreichende Führung der Vorrichtung (10) beim Einstich zu gewährleisten, ist die Vorrichtung (10) in eine Einschubhülse (115) eingebracht, die verhindert, dass die Vorrichtung (10) beim Einschieben in den Körper abknickt oder verrutscht. Ein Kontaktierungselement (114), welches auf der Hautoberfläche befestigt ist, dient zur Kontaktierung des Kontaktierungsbereiches (118) nach Einschieben der Vorrichtung (10) in den Körper, wie dies in Figur 4a dargestellt ist. In dem Kontaktierungsbereich (118) münden die elektrischen Verbindungen von den Zuleitungen (3) aus dem Sensorbereich (2a) und werden von den Kontakten (113) des Kontaktierungselementes (114) kontaktiert. Dieser Kontaktierungsbereich (118) wird durch Dichtungselemente (117), die sich in dem Kontaktierungselement (114) befinden gegen Eindringen von Flüssigkeit geschützt. In dem Kontaktierungselement (114) kann sich eine Stromquelle zur Versorgung des Sensors (4) befinden, sowie ein Übertragungsmodul zum Übertragen von Daten an ein weiteres Gerät, was hier nicht dargestellt ist. Durch die äußere Abstützung der Vorrichtung (10) bei der Applikation ist kein Zugdraht (5) zusätzlich zum Butterfly (14) zum Spannen des Butterfly (14) und damit der Segmente notwendig.

### Bezugszeichenliste

- 1: distaler Bereich
- 1a: Nadelspitze
- 2: Segmentbereich
- 2a: Sensorbereich
- 2b: mittlerer Segmentbereich
- 2c: proximaler Bereich
- 2d: Segment
- 3: Zuleitung
- 4: Sensor
- 5: Zugdraht
- 6: Gewebe
- 7: Flüssigkeitsstopp
- 8: Membran
- 9: Hülle
- 9a: erster Teil Hebeschräge
- 9b: zweiter Teil Hebeschräge
- 10: Vorrichtung zum Einbringen
- 12: Reagenzien
- 14: Zugvorrichtung (Butterfly)
- 14a: erster Flügel
- 14b: zweiter Flügel
- 22: Perforation
- 111: erste Hebeschräge
- 112: zweite Hebeschräge
- 113: Kontakt
- 114: Kontaktierungselement
- 115: Einschubhülse
- 116: Applikator
- 117: Dichtung
- 118: Kontaktierungsbereich

## Patentansprüche

1. Eine Einbringvorrichtung (10) zum Einbringen einer medizinischen Vorrichtung in einen Körper mit:
- einem distalen Bereich (1), der einen Spitzenbereich (1a) zum Erzeugen einer Hautöffnung aufweist, und
- einem Segmentbereich (2), der mindestens zwei Teilbereiche (2d) beinhaltet, und
- die mindestens zwei Teilbereiche zum Einbringen der Vorrichtung in einem rigiden Zustand mindestens miteinander und mit dem distalen Bereich im Wesentlichen starr verbunden sind und die mindestens zwei Teilbereiche nach dem Einbringen der Vorrichtung in einem flexiblen Zustand gegeneinander beweglich sind, wobei die mindestens zwei Teilbereiche im rigiden Zustand über ein Fixierungsmittel miteinander und mit dem distalen Bereich starr verbunden sind,
**dadurch gekennzeichnet, dass** das Fixierungsmittel im Körper zumindest teilweise auflösbar und/oder quillbar ist und **dadurch** den flexiblen Zustand ermöglicht.

2. Vorrichtung nach Anspruch 1, **dadurch gekennzeichnet, dass** der rigide Zustand durch ein formgebendes Element (5) bewirkt wird.

3. Vorrichtung nach Anspruch 1 oder 2, **dadurch gekennzeichnet, dass** die Einbringvorrichtung mindestens in einem Teil des distalen Bereiches und/oder mindestens in einem Teil der Teilbereiche hohl ist.

4. Vorrichtung nach einem der vorhergehenden Ansprüche, **dadurch gekennzeichnet, dass** die mindestens zwei Teilbereiche aus im Wesentlichen starren Segmenten bestehen.

5. Vorrichtung nach Anspruch 4, **dadurch gekennzeichnet, dass** die Segmente durch ein formgebendes Element zusammengehalten werden.

6. Vorrichtung nach Anspruch 5, **dadurch gekennzeichnet, dass** das formgebende Element ein Zugdraht (5) ist.

7. Vorrichtung nach Anspruch 6, **dadurch gekennzeichnet, dass** der Zugdraht sich bei Temperaturerhöhung zusammenzieht.

8. Vorrichtung nach einem der Ansprüche 6 oder 7, **dadurch gekennzeichnet, dass** der Zugdraht durch eine Zugvorrichtung gestrafft wird.

9. Vorrichtung nach einem der vorhergehenden Ansprüche, **dadurch gekennzeichnet, dass** mindestens die mindestens zwei Teilabschnitte mit einer Membran (8) überzogen sind.

10. Vorrichtung nach Anspruch 9, **dadurch gekennzeichnet, dass** die Membran flexibel ist.

11. Vorrichtung nach einem der Ansprüche 9 oder 10, **dadurch gekennzeichnet, dass** die Membran das Fixierungsmittel enthält.

## Claims

1. An introducing device (10) for introducing a medical device into a body comprising:
- a distal region (1) which has a tip region (1a) for generating an opening in the skin, and
- a segment region (2) having at least two sections (2d), and
- the at least two sections are essentially rigidly connected to at least one another and to the distal region in a rigid state for introducing the device and the at least two sections are movable relative to one another in a flexible state after the device has been introduced, wherein in the rigid state the at least two sections are rigidly connected to one another and to the distal region by an immobilizing agent, **characterized in that** the immobilizing agent can at least partially dissolve and/or swell in the body and thus enable the flexible state.

2. Device according to claim 1, **characterized in that** the rigid state is brought about by a forming element (5).

3. Device according to claim 1 or 2, **characterized in that** the introducing device is hollow at least in a part of the distal region and/or at least in a part of the sections.

4. Device according to one of the previous claims, **characterized in that** the at least two sections consist of essentially rigid segments.

5. Device according to claim 4, **characterized in that** the segments are held together by a forming element.

6. Device according to claim 5, **characterized in that** the forming element is a pull wire (5).

7. Device according to claim 6, **characterized in that** the pull wire contracts when the temperature increases.

8. Device according to one of the claims 6 or 7, **characterized in that** the pull wire is tightened by a pulling device.

9. Device according to one of the previous claims, **characterized in that** the at least two sections are covered with a membrane (8).

10. Device according to claim 9, **characterized in that** the membrane is flexible.

11. Device according to one of the claims 9 or 10, **characterized in that** the membrane contains the immobilizing agent.

## Revendications

1. Dispositif d'insertion (10) pour introduire un dispositif médical dans un corps avec :
- une région distale (1) qui présente une région pointue (1a) pour produire une ouverture cutanée, et
- une région segmentée (2) qui renferme au moins deux régions partielles (2d) et
- lesdites au moins deux régions partielles pour introduire le dispositif dans un état rigide, sont essentiellement reliées solidement au moins l'une à l'autre et à la région distale, et lesdites au moins deux régions partielles après l'introduction du dispositif dans un état souple sont mobiles l'une par rapport à l'autre, lesdites au moins deux régions partielles dans l'état rigide sont reliées solidement l'une à l'autre et à la région distale par un moyen de fixation, **caractérisé en ce que** le moyen de fixation dans le corps est au moins partiellement soluble et/ou gonflable et permet ainsi d'obtenir l'état souple.

2. Dispositif selon la revendication 1, **caractérisé en ce que** l'état rigide est induit par un élément (5) donnant la forme.

3. Dispositif selon la revendication 1 ou 2, **caractérisé en ce que** le dispositif d'insertion est creux au moins dans une partie de la région distale et/ou au moins dans une partie des régions partielles.

4. Dispositif selon l'une quelconque des revendications précédentes, **caractérisé en ce que** lesdites au moins deux régions partielles se composent essentiellement de segments rigides.

5. Dispositif selon la revendication 4, **caractérisé en ce que** les segments sont maintenus ensemble par un élément donnant la forme.

6. Dispositif selon la revendication 5, **caractérisé en ce que** l'élément donnant la forme est un fil de traction (5).

7. Dispositif selon la revendication 6, **caractérisé en ce que** le fil de traction se contracte lors d'une augmentation de la température.

8. Dispositif selon l'une quelconque des revendications 6 ou 7, **caractérisé en ce que** le fil de traction est tendu par un dispositif de traction.

9. Dispositif selon l'une quelconque des revendications précédentes, **caractérisé en ce qu'**au moins lesdites au moins deux sections partielles sont recouvertes par une membrane (8).

10. Dispositif selon la revendication 9, **caractérisé en ce que** la membrane est souple.

11. Dispositif selon l'une quelconque des revendications 9 ou 10, **caractérisé en ce que** la membrane contient le moyen de fixation.
